(19) Europäisches Patentamt
European Patent Office
Office européen des brevets

(11) **EP 3 150 111 B1**

(12) **EUROPEAN PATENT SPECIFICATION**

(45) Date of publication and mention
of the grant of the patent:
**08.04.2020 Bulletin 2020/15**

(51) Int Cl.:
***A61B 3/15*** *(2006.01)*

(21) Application number: **16191362.9**

(22) Date of filing: **29.09.2016**

(54) **OPHTHALMIC APPARATUS AND CONTROL PROGRAM FOR THE OPHTHALMIC APPARATUS**

OPHTHALMISCHE VORRICHTUNG UND STEUERUNGSPROGRAM FÜR DIE OPHTHALMISCHE VORRICHTUNG

APPAREIL OPHTALMIQUE ET PROGRAMME DE COMMANDE DE L'APPAREIL OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.09.2015 JP 2015193531**

(43) Date of publication of application:
**05.04.2017 Bulletin 2017/14**

(73) Proprietor: **Nidek Co., Ltd.**
**Gamagori-shi, Aichi 443-0038 (JP)**

(72) Inventor: **ARIKAWA, Toru**
**Gamagori-shi, Aichi 443-0038 (JP)**

(74) Representative: **Prüfer & Partner mbB**
**Patentanwälte · Rechtsanwälte**
**Sohnckestraße 12**
**81479 München (DE)**

(56) References cited:
**EP-A1- 2 085 023    EP-A2- 2 505 127**
**JP-A- 2013 027 537    US-A1- 2001 024 264**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

This disclosure relates to an ophthalmic apparatus for examining an eye of an examinee and a control program for the ophthalmic apparatus.

As conventional ophthalmic apparatuses, for example, there are known an eye refractive power measuring apparatus, a corneal curvature measuring apparatus, an intraocular pressure measuring apparatus, a fundus camera, an OCT, an SLO, and so on. Those ophthalmic apparatuses are generally configured to move an optometry unit up and down, right and left, and back and forth directions with respect to an examinee's eye by operation of an operation member, such as a joystick, to align the optometry unit to a predetermined position with respect to the examinee's eye (see Patent Document 1).

One of the conventional ophthalmic apparatuses is proposed as an apparatus configured to make positioning of an optometry unit with respect to an examinee's eye based on a photographed image of a face of an examinee (see Patent Document 2).

EP 2 085 023 A1 discloses an ophthalmic apparatus according to the preamble of claim 1.

Related Art Documents

Patent Documents

Patent Document 1: Japanese unexamined patent publication application No. 2013-066760
Patent Document 2: Japanese unexamined patent publication application No. H10(1998)-216089

SUMMARY

However, in the conventional apparatus disclosed in Patent Document 2, a face photographing unit for photographing the examinee's face may be complicated in structure.

The present disclosure has been made to address the above problems and has a purpose to provide an ophthalmic apparatus capable of automatic alignment with a simple structure and a control program for the ophthalmic apparatus.

The above mentioned object of the present invention is achieved by the ophthalmic apparatus according to claim 1 and the control program for an ophthalmic apparatus according to claim 8.

Further developments of the present invention are given in the dependent claims.

BRIEF DESCRIPTION OF THE DRAWINGS

FIG. 1 is a schematic diagram showing an outer appearance of a present example;
FIG. 2 is a block diagram showing a control system of the present example;
FIG. 3 is a schematic diagram showing optical systems of the present example;
FIG. 4 is a flowchart showing control operations in the present example;
FIG. 5 is a diagram to explain a positional relationship between a photographing unit and an examinee's eye in the present example;
FIG. 6 is a diagram to explain a moving manner of an optometry unit;
FIG. 7 is a diagram to explain a moving manner of an optometry unit; and
FIG. 8 is a diagram to explain a modified example of the moving manner of the optometry unit.

DETAILED DESCRIPTION OF THE EXEMPLARY EMBODIMENTS

An embodiment will be briefly described below referring to the accompanying drawings. An ophthalmic apparatus (e.g., an ophthalmic apparatus 1) of the present embodiment is used for example to examine an eye of an examinee. For instance, this ophthalmic apparatus may measure eye refractive power, corneal curvature, intraocular pressure, and other conditions of the examinee's eye and also may photograph or capture images of an anterior segment, a fundus, and other portions of the examinee's eye. The ophthalmic apparatus is provided with for example an optometry unit (e.g., an optometry unit 2), a drive unit (e.g., a drive unit 4), a first photographing unit (e.g., a face photographing unit 3), a control unit (e.g., a control unit 70), and others. The optometry unit examines the examinee's eye, for example. The optometry unit may be provided with for example at least one of various examination optical systems, such as an eye refractive power measuring optical system, a corneal curvature measuring optical system, an intraocular pressure measuring optical system, a fundus photographing optical system, and a tomographic image photographing optical system. The drive unit relatively moves the optometry unit three-dimensionally with respect to the examinee's eye. The first photographing unit photographs for example the face of the examinee including at least one of his/her right and left eyes.

The control unit controls driving of the drive unit. This control unit restricts a drive range of the drive unit based on a two-dimensional position of the examinee's eye on a first image and information on the first photographing unit. Accordingly, the optometry unit can be efficiently aligned with the examinee's eye. The first image is an image photographed by the first photographing unit. The two-dimensional position of the examinee's eye on the first image is specified by image processing on the first image. The information on the first photographing unit includes positional information of the first photographing unit, and may also include a camera parameter of the first photographing unit.

It is to be noted that the control unit is also configured to restrict the drive range of the drive unit based on a three-dimensional relative direction between the examinee's eye and the first photographing unit, the three-dimensional relative direction being calculated based on the two-dimensional position of the examinee's eye on the first image and the information on the first photographing unit.

The optometry unit is provided with a second photographing unit (e.g., an anterior segment photographing optical system 60). The second photographing unit is configured to observe an anterior segment. This second photographing unit is provided as a separate unit from the first photographing unit. The second photographing unit is different from the first photographing unit. For instance, the second photographing unit is placed in a position different from the first photographing unit.

When the positional relationship between the examinee's eye and the optometry unit is obtained by image processing on the second image photographed by the second photographing unit, the control unit controls the drive unit based on the obtained positional relationship to thereby perform alignment (positioning) of the optometry unit with respect to the examinee's eye. Furthermore, when the positional relationship between the examinee's eye and the optometry unit is not obtained from the second image, the control unit restricts the drive range of the drive unit based on the two-dimensional position of the examinee's eye on the first image specified by the image processing on the first image and the information on the first photographing unit. The control unit searches for the examinee's eye within the restricted drive range to make the second photographing unit efficiently perform alignment.

The control unit is configured to calculate a straight line passing through the position of the first photographing unit and has a directional vector defined by a three-dimensional relative direction. In this case, the control unit is configured to restrict the drive range so that at least part of the straight line is included in an alignment allowable region provided by the second photographing unit. For instance, the control unit may control the drive unit within the restricted drive range to move the optometry unit to the position where the examinee's eye is included in the alignment allowable region provided by the second photographing unit. Herein, the alignment allowable region may be for example at least part of a photographing range of the second photographing unit. For instance, the alignment allowable region is a region enabling determination of a three-dimensional position of the examinee's eye from an anterior segment image photographed in the photographing range of the second photographing unit. For instance, when the examinee's eye is located within the alignment allowable region, the control unit can obtain the three-dimensional position of the examinee's eye from the anterior segment image photographed by the second photographing unit.

The control unit may also be configured to control the drive unit to move the alignment allowable region of the second photographing unit along the aforementioned straight line. In this manner, the control unit can smoothly move the alignment allowable region of the second photographing unit to the position of the examinee's eye.

The control unit may also be configured to update a restrictive condition of the drive range based on a new first image photographed by the first photographing unit. This enables smooth alignment even when the examinee's eye moves or the visual line direction of the examinee's eye changes during alignment of the optometry unit.

The first photographing unit may be provided in the optometry unit. In this case, the first photographing unit is moved together with the second photographing unit by the drive unit. That is, the relative positional relationship between the first photographing unit and the second photographing unit is constant.

The present apparatus may be further provided with a face support unit (e.g., a face support unit 9) for supporting the face of the examinee. In this case, the first photographing unit is provided in a position where the relative positional relationship between the face support unit and the first photographing unit is constant.

The control unit may be configured to detect a pupil of the examinee's eye from the first image photographed by the first photographing unit and restrict the drive range of the drive unit based on the two-dimensional position of the pupil on the first image and the information on the first photographing unit.

The present apparatus may be further provided with a target projecting optical system (e.g., an alignment target projecting optical system 50). The target projecting optical system projects for example an alignment target (mark) onto a corneal of the examinee's eye. In this case, for instance, the control unit may be configured to detect the position of the alignment target from the second image photographed by the second photographing unit. Based on this position of the alignment target, the control unit may also perform alignment of the optometry unit with respect to the examinee's eye.

Further, the control unit may be configured to cause the ophthalmic apparatus to execute a program of controlling the ophthalmic apparatus (an "ophthalmic apparatus control program"), the program having been stored in a storage unit (e.g., a storage unit 74). This ophthalmic apparatus control program includes for example a photographing step, a drive step, and an optometry step. The photographing step is for example a step of photographing the face of an examinee including at least one of his/her right and left eyes by the photographing unit. The drive step is for example a step of relatively moving the optometry unit three-dimensionally with respect to the examinee's eye based on the two-dimensional position of the examinee's eye on the first image photographed in the photographing step, the two-dimensional position being specified by image processing on the first image, and the information on the photographing unit. The optometry step is a step of examining the examinee's eye.

The ophthalmic apparatus of the present embodiment configured as above preferentially drives the drive range calculated based on the two-dimensional position of the examinee's eye on the first image and the information on the first photographing unit and thereby can rapidly detect the examinee's eye, thus achieving shortened alignment time.

Furthermore, as described above, when the positional relationship between the examinee's eye and the optometry unit is obtained by the image processing on the second image photographed by the second photographing unit, the ophthalmic apparatus of the present embodiment controls the drive unit based on the obtained positional relationship to perform alignment (positioning) of the optometry unit with respect to the examinee's eye. In contrast, when the positional relationship between the examinee's eye and the optometry unit is not obtained from the second image, the ophthalmic apparatus restricts the drive range of the drive unit based on the two-dimensional position of the examinee's eye on the first image specified by the image processing on the first image and the information on the first photographing unit. The ophthalmic apparatus of the present embodiment configured to repeat this processing can finally efficiently perform alignment of the optometry unit based on the second image.

<Examples>

The ophthalmic apparatus of this disclosure will be explained referring to the drawings. In the following description, an eye refractive power measuring apparatus is explained as an example of the ophthalmic apparatus. However, this disclosure is also applicable to other ophthalmic apparatuses, such as a corneal curvature measuring apparatus, an intraocular pressure measuring apparatus, a fundus camera, an OCT (Optical Coherence Tomography), an SLO (Scanning Laser Ophthalmoscope).

The ophthalmic apparatus of the present example measures an eye refractive power of the examinee's eye in an objective manner. For instance, the ophthalmic apparatus of the present example may be configured to measure each eye separately or measure both eyes simultaneously (binocular vision). The ophthalmic apparatus mainly includes for example an optometry unit, a photographing unit, a drive unit, and a control unit.

<Outer Appearance>

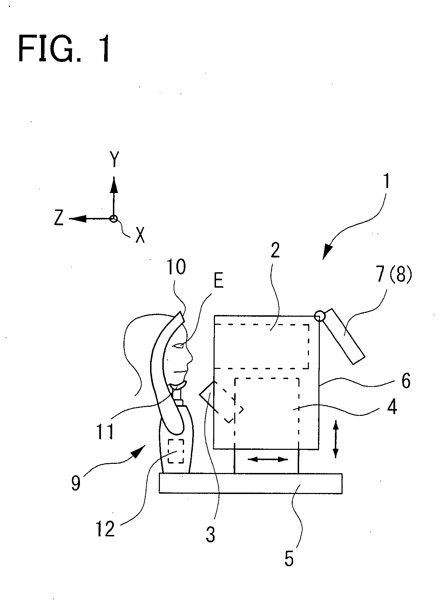

The outer appearance of the ophthalmic apparatus will be explained referring to FIG. 1. As shown in FIG. 1, the ophthalmic apparatus 1 of the present example mainly includes the optometry unit 2, the face photographing unit 3, and the drive unit 4. The optometry unit 2 examines an examinee's eye E. The optometry unit 2 may be provided with for example an optical system(s) for measuring eye refractive power, corneal curvature, intraocular pressure, and so on, of the examinee's eye E. The optometry unit 2 may further include an optical system for photographing an anterior segment, a fundus, and so on of the examinee's eye E. In the present example, the optometry unit 2 for measuring refractive power is exemplified. The face photographing unit 3 photographs the examinee's face, for example. The face photographing unit 3 photographs the examinee's face including at least one of his/her right and left eyes. The drive unit 4 moves the optometry unit 2 and the face photographing unit 3 with respect to a base table 5 in up and down, right and left, and back and forth directions (in a three-dimensional direction).

Furthermore, the ophthalmic apparatus 1 of the present example may also include for example a housing 6, a display unit 7, an operation unit 8, the face support unit 9, and others. For instance, the housing 6 houses the optometry unit 2, the face photographing unit 3, the drive unit 4, and others. The display unit 7 displays for example an observation image of the examinee's eye E, a measured result, and others. For instance, the display unit 7 may be provided integrally with the apparatus 1 or separately from the apparatus 1. The ophthalmic apparatus 1 may also be provided with the operation unit 8. This operation unit 8 is used for operations to input various settings of the apparatus 1 and start measurement. The operation unit 8 is operated by an examiner to enter various operational commands. The operation unit 8 may be for example various types of human interfaces, such as a touch panel, a joystick, a mouse, a keyboard, a track ball, and a button. The face support unit 9 may be provided for example with a forehead rest 10 and a chin rest 11. The chin rest 11 may be moved in up and down directions by driving of a chin-rest drive unit 12.

<Control System>

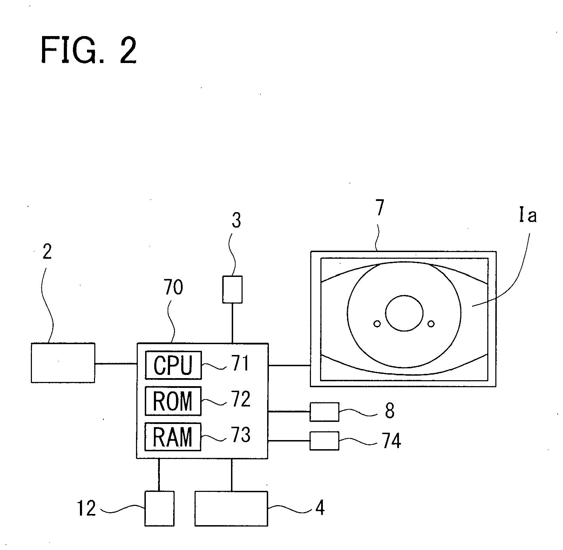

As shown in FIG. 2, the present apparatus 1 includes the control unit 70. This control unit 70 takes charges of various controls of the present apparatus 1. The control unit 70 includes for example a general CPU (Central Processing Unit) 71, a flash ROM 72, a RAM 73, and others. For instance, the flash ROM 72 has stored an ophthalmic apparatus control program of controlling the ophthalmic apparatus 1, default values, and others. For instance, the RAM 73 temporarily stores various information. The control unit 70 is connected to the optometry unit 2, the face photographing unit 3, the drive unit 4, the display unit 7, the operation unit 8, the chin-rest drive unit 12, the storage unit (e.g., a non-volatile memory) 74, and others. The storage unit 74 is a non-transitory storage medium capable of retaining stored contents even if power supply is shut off. For example, a hard disc drive, a removable USB flash memory, and others may be used as the storage unit 74.

<Face Photographing Unit>

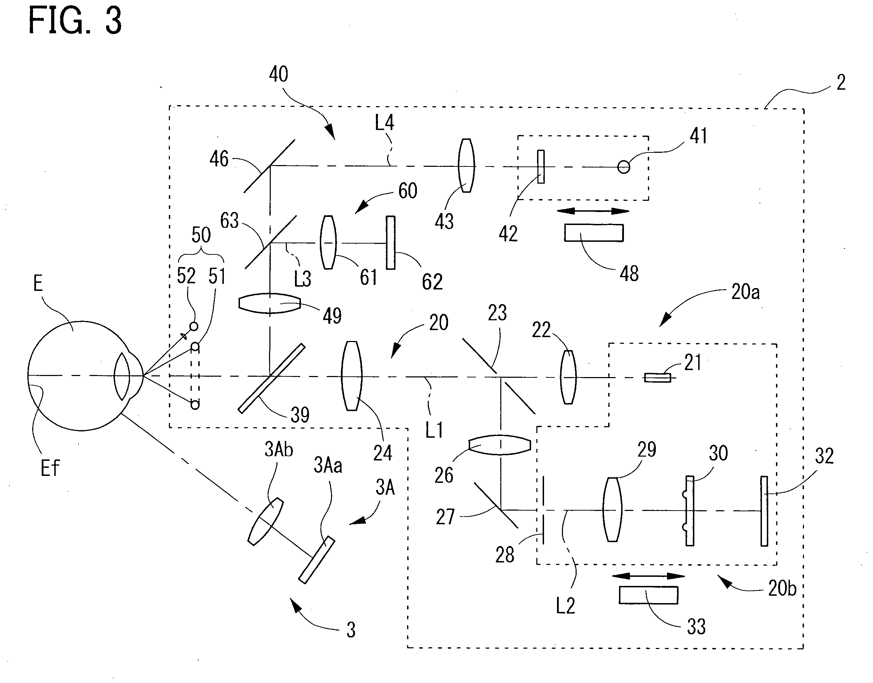

The face photographing unit 3 photographs the face of the examinee including at least one of his/her right and left eyes. For instance, as shown in FIG. 3, the face photographing unit 3 of the present example is provided with a photographing optical system 3A for photographing the examinee's face. The photographing optical system 3A mainly includes for example an imaging element 3Aa and an imaging lens 3Ab. The face photographing unit 3 is for example a non-telecentric optical system. Thus, the apparatus does not need to include any telecentric lens and thus can provide a simple structure. The apparatus can further provide a wider photographing range than the telecentric optical system.

The face photographing unit 3 of the present example is moved together with the optometry unit 2 by the drive unit 4. Of course, for instance, the face photographing unit 3 may be fixed to the base table 5 and held against movement.

In FIG. 1, the face photographing unit 3 is placed under the optometry unit 2. However, the position of the face photographing unit 3 is not limited thereto. For instance, the face photographing unit 3 may be provided above or lateral to the optometry unit 2. They may also be provided so that a measurement optical axis of the optometry unit 2 becomes coaxial with a photographing optical axis of the face photographing unit 3. As an alternative, the face photographing unit 3 may be placed independently from movement of the optometry unit 2. For instance, the face photographing unit 3 may be provided on the base table 5 so as to be three-dimensionally driven, so that face photographing unit 3 is driven in a three-dimensional manner by another drive unit (a second drive unit), which is different from the drive unit (a first drive unit) 4. Of course, the first drive unit for moving the optometry unit 2 may serve as the second drive unit for moving the photographing unit 3 as in the present example.

<Optometry Unit>

The optometry unit 2 performs measurement, examination, photographing, and so on, of the examinee's eye E. The optometry unit 2 may be provided with for example a measurement optical system for measuring refractive power of the examinee's eye E. As shown in FIG. 3, for instance, the optometry unit 2 may include a measurement optical system 20, a fixation target presenting optical system 40, an alignment target projecting optical system 50, and an anterior segment photographing optical system 60.

The measurement optical system 20 includes a projecting optical system (a light projecting optical system) 20a and a light receiving optical system 20b. The projecting optical system 20a projects a light flux to a fundus Ef through a pupil of the examinee's eye E. The light receiving optical system 20b extracts a reflection light flux (fundus reflection light) as ring-shaped light reflected from the fundus Ef through a pupil and its surrounding area and images a ring-shaped fundus reflection image to be mainly used for measurement of refractive power.

The projecting optical system 20a includes, on its optical axis L1, a measurement light source 21, a relay lens 22, a hole mirror 23, and an objective lens 24. The light source 21 projects a spot-shaped source image on the fundus Ef through the relay lens 22 to the objective lens 24 and a pupil central area. The light source 21 is moved along the optical axis L1 by a movement mechanism 33. The hole mirror 23 is provided with an aperture allowing a light flus emitted from the light source 21 via the relay lens 22 to pass through. The hole mirror 23 is placed in an optically conjugate position with the pupil of the examinee's eye E.

The light receiving optical system 20b shares the hole mirror 23 and the objective lens 24 with the projecting optical system 20a. The light receiving optical system 20b further includes a relay lens 26 and a total reflection mirror 27. Moreover, the light receiving optical system 20b includes a light-receiving diaphragm 28, a collimator lens 29, a ring lens 30, and an imaging element 32, on an optical axis L2 corresponding to a reflection direction of the hole mirror 23. As the imaging element 32, a two-dimensional light-receiving element, such as an area CCD, can be used. The light-receiving diaphragm 28, the collimator lens 29, the ring lens 30, and the imaging element 32 are moved integrally with the measurement light source 21 of the projecting optical system 20a in the optical axis L2 direction by the movement mechanism 33. When the light source 21 is placed in an optically conjugate position with the fundus Ef by the movement mechanism 33, the light-receiving diaphragm 28 and the imaging element 32 are also placed in each optically conjugate position with the fundus Ef.

The ring lens 30 is an optical element to form the fundus reflection light directed from the objective lens 24 via the collimator lens 29 into a ring shape. The ring lens 30 has a ring-shaped lens part and a light-shielding part. Further, when the light-receiving diaphragm 28 and the imaging element 32 are placed in each optically conjugate position with the fundus Ef, the ring lens 30 is disposed in an optically conjugate position with a pupil of the examinee's eye E. The imaging element 32 receives the ring-shaped fundus reflection light (hereinafter, called a ring image) passing through the ring lens 30. The imaging element 32 outputs image information on the received ring image to the CPU 71. Consequently, the CPU 71 displays the ring image on the display unit 7 and calculates the refractive power based on the ring image, and so on.

In the present example, furthermore, as shown in FIG. 3, a dichroic mirror 39 is placed between the objective lens 24 and the examinee's eye E. The dichroic mirror 39 transmits the light emitted from the light source 21 and the fundus reflection light deriving from the light from the light source 21. The dichroic mirror 39 directs a light flux from a fixation target presenting optical system 40 described later to the examinee's eye E. Further, the dichroic mirror 39 reflects the light reflected from an anterior segment, deriving from an alignment target projecting optical system 50 described later, to direct this anterior segment reflection light to the anterior segment photographing optical system 60.

As shown in FIG. 3, in front of the examinee's eye E, the alignment target projecting optical system 50 is placed. This alignment target projecting optical system 50 mainly projects a target image to be used for positioning (alignment) of the optical system with respect to the examinee's eye E onto the anterior segment. The alignment target projecting optical system 50 includes a ring target projecting optical system 51 and a target projecting optical system 52. The ring target projecting optical system 51 projects diffusion light onto a cornea of the examinee's eye E to project thereon a ring target. In the ophthalmic apparatus 1 of the present example, the ring target projecting optical system 51 is also used for anterior segment illumination to illuminate the anterior segment of the examinee's eye E. The target projecting optical system 52 projects parallel light onto a cornea of the examinee's eye E to project thereon an infinite target.

The fixation target presenting optical system 40 includes a light source 41, a fixation target 42, and a relay lens 43 on an optical axis L4 corresponding to a reflection direction of the reflection mirror 46. The fixation target 42 is used to make the examinee's eye E fixate thereat during objective refractive power measurement. For instance, when illuminated with light of the light source 41, the fixation target 42 is presented to the examinee's eye E.

The light source 41 and the fixation target 42 are integrally moved by a drive mechanism 48 in a direction of the optical axis L4. A presenting position (a presenting distance) of the fixation target may be changed by movement of the light source 41 and the fixation target 42. This enables performing refractive power measurement with fogging applied to the examinee's eye E.

The anterior segment photographing optical system 60 includes an imaging lens 61 and an imaging element 62 on an optical axis L3 corresponding to a reflection direction of a half mirror 63. The imaging element 62 is placed in an optically conjugate position with the anterior segment of the examinee's eye E. The imaging element 62 captures an image of the anterior segment illuminated by the ring target projecting optical system 51. Output from the imaging element 62 is input to the CPU 71. As a result, an anterior segment image Ia of the examinee's eye E imaged by the imaging element 62 is displayed on the display unit (see FIG. 2). The imaging element 62 captures an alignment target image (a ring target and an infinite target in the present example) formed on a cornea of the examinee's eye E by the alignment target projecting optical system 50. Accordingly, the CPU 71 can detect an alignment target image based on an imaging result of the imaging element 62. The CPU 71 also can determine whether an alignment state is appropriate or not based on the position at which the alignment target image is detected.

<Control Method>

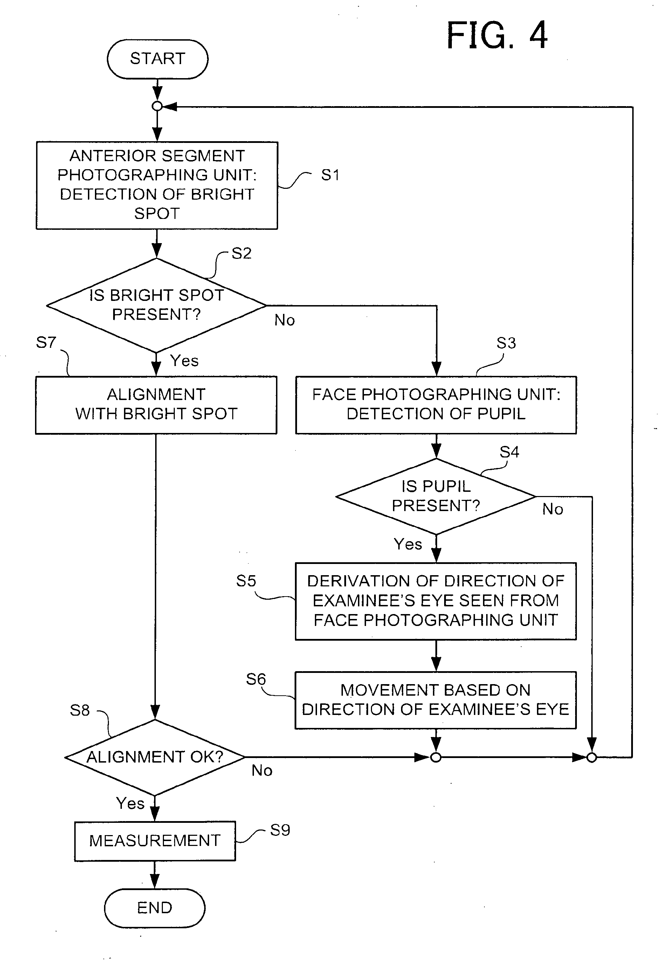

Control operation of the present apparatus 1 is explained below. The present apparatus 1 for example automatically performs positioning (alignment) between the optometry unit 2 and the examinee's eye E for examination of the examinee's eye E. In the present example, for instance, detection of a bright spot (a luminescent spot) by the anterior segment photographing optical system 60 and detection of an eye by the face photographing unit 3 are performed in parallel. When the CPU 71 detects the bright spot (alignment target) from an anterior segment image Ia (see FIG. 2), the CPU 71 can calculate an alignment position from that detected position. When the pupil can be detected from the face image Pi, the direction in which the examinee's eye E is located ("direction of the examinee's eye E") when seen from the face photographing unit 3 in a real space is found based on a coordinate of the examinee's eye of the face image Pi and the information on the photographing unit 3 (e.g., positional information, camera parameter, and others). The present example utilizes those two types of data. Until the bright spot is detected by the anterior segment photographing optical system 60, the optometry unit 2 is moved based on the direction E1 of the examinee's eye E, which has been found from the face image Pi. After the bright spot has been detected by the anterior segment photographing optical system 60, for example, the CPU 71 performs alignment with the bright spot and then, upon completion of the alignment, carries out the measurement. The control flow is explained referring to a flowchart of FIG. 4.

(S1: Detection of bright spot)

The CPU 71 firstly determines whether or not a target (e.g., a bright spot) projected onto the anterior segment of the examinee's eye E by the target projecting optical system 50 is detected. For instance, the CPU 71 detects the bright spot by processing the image photographed by the anterior segment photographing optical system 60. For instance, the CPU 71 obtains information on the bright spot in the image Ia photographed by the anterior segment photographing optical system 60.

(S2: Determination of presence/absence of bright spot)

Successively, the CPU 71 determines for instance whether or not the bright spot is present in the anterior segment image Ia based on a detection result in step S1. When the bright spot is present, for instance, the CPU 71 advances the processing to step S7. In the absence of the bright spot, the CPU 71 goes to the processing in step S3. For instance, the CPU 71 can determine that the bright spot is detected when the luminance equal to or higher than a threshold is detected, while determine that the bright spot is not detected when the luminance equal to or higher than a threshold is not detected.

(S3: Detection of pupil)

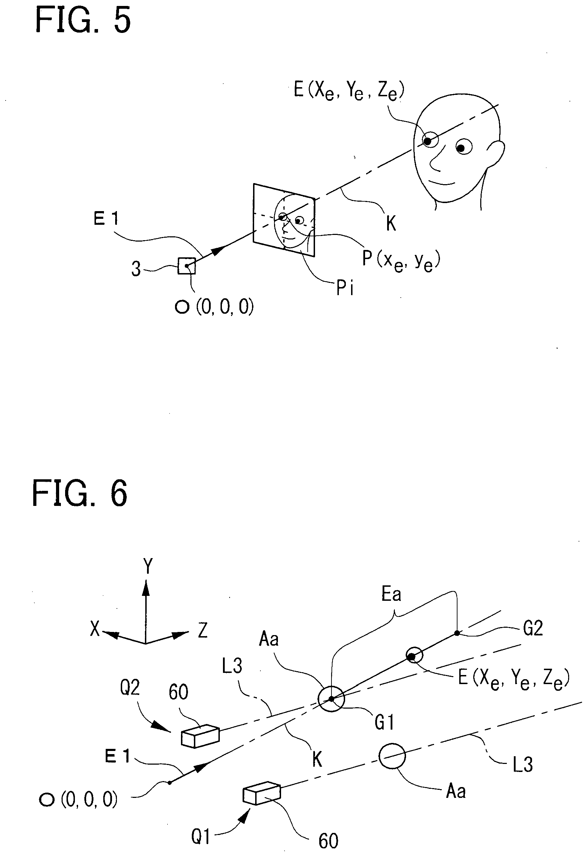

When the bright spot is not detected in step S2, the CPU 71 obtains an image of the face including the examinee's eye E photographed by the face photographing unit 3. The CPU 71 analyzes the obtained face image Pi (see FIG. 5) and detects the examinee's eye E (e.g., pupil). For example, the CPU 71 may be configured to detect an edge of the face image and detect the pupil of the examinee's eye E based on the shape of the edge and others.

(S4: Determination of presence/absence of pupil)

When the pupil is detected in step S3, the CPU 71 goes to the processing in step S5. When the pupil is not detected, the CPU 71 returns to the processing in step S1.

(S5: Derivation of direction of examinee's eye E seen from face photographing unit 3)

When the pupil is detected in step S4, the CPU 71 calculates the direction E1 of the examinee's eye E seen from the face photographing unit 3 (see FIG. 5). The direction E1 of the examinee's eye E may be for example a three-dimensional direction (e.g., space vector). The CPU 71 firstly calculates a coordinate P $(x_e, y_e)$ of the examinee's eye E (e.g., pupil) on the face image Pi. For instance, the coordinate of the examinee's eye E may be calculated by analysis of the luminance of the face image Pi, the edge, and others.

Herein, the three-dimensional coordinate of the examinee's eye E seen from the face photographing unit 3 is assumed to be E = $(X_e, Y_e, Z_e)$, wherein the origin is the face photographing unit 3. At that time, the direction E1 of the examinee's eye E seen from the face photographing unit 3 is E1's unit vector (E1/|E1|). The CPU 7 determines this direction E1 of the examinee's eye E, for example.

When the examinee's eye is projected at the coordinate P$(x_e, y_e)$ on the face image Pi, a relationship between E1 and $(x_e, y_e)$ is given by the following expression (1):

$$ \mathrm{h} \begin{pmatrix} x_e \\ y_e \\ 1 \end{pmatrix} = J \begin{pmatrix} X_e \\ Y_e \\ Z_e \end{pmatrix} \ , \ J = \begin{pmatrix} f_x & s & c_x \\ 0 & f_y & c_y \\ 0 & 0 & 1 \end{pmatrix} \qquad (1) $$

wherein a matrix J is an internal parameter matrix of the face photographing unit 3, $f_x$ and $f_y$ are focal distances, s is skew distortion, and $(c_x, c_y)$ is an optical center on the image. These values are obtained in advance by calibration of the face photographing unit 3.

The expression (1) is expanded and substituted to establish the following simultaneous equations (2).

$$\begin{cases} x_e = f_x \dfrac{X_e}{Z_e} + s\,\dfrac{Y_e}{Z_e} + c_x \\[2mm] y_e = f_y \dfrac{Y_e}{Z_e} + c_y \end{cases} \qquad (2)$$

The simultaneous equations (2) are solved to find $X_e/Z_e$ and $Y_e/Z_e$, so that a ratio $X_e:Y_e:Z_e$ is found. That is, the direction E1 of the examinee's eye E seen from the face photographing unit 3 is obtained. In this manner, the CPU 71 may also be configured to determine the direction E1 of the examinee's eye E seen from the face photographing unit 3.

(S6: Movement based on direction of examinee's eye)

The CPU 71 sets a drive range of the drive unit 4 based on the direction E1 of the detected examinee's eye E and moves the optometry unit 2 within the set drive range. For instance, the CPU 71 shifts the alignment allowable region Aa by the anterior segment photographing optical system 60 onto the straight line K connecting the face photographing unit 3 and the examinee's eye. Herein, the alignment allowable region Aa is for example a region enabling detection of a three-dimensional position of the examinee's eye E from the anterior segment image Ia photographed in a certain position by the anterior segment photographing optical system 60. A positional relationship between an origin O of the face photographing unit 3 and the alignment allowable region Aa by the anterior segment photographing optical system 60 is known because of the design of the apparatus. Thus, the CPU 71 can cause the drive unit 4 to move the alignment allowable region Aa onto the straight line K. For instance, the CPU 71 moves the anterior segment photographing optical system 60 from a position Q1 to a position Q2 so that the straight line K is included in the alignment allowable region Aa as shown in FIG. 6. Herein, the position Q2 is a location causing a position G1, on the straight like K, closest to the origin O within a measurable range Ea allowing measurement using the optometry unit 2, to coincide with the gravity point of the alignment allowable region Aa. The measurable range Ea is determined for example based on a working distance of the optometry unit 2 and the drive range of the drive unit 4.

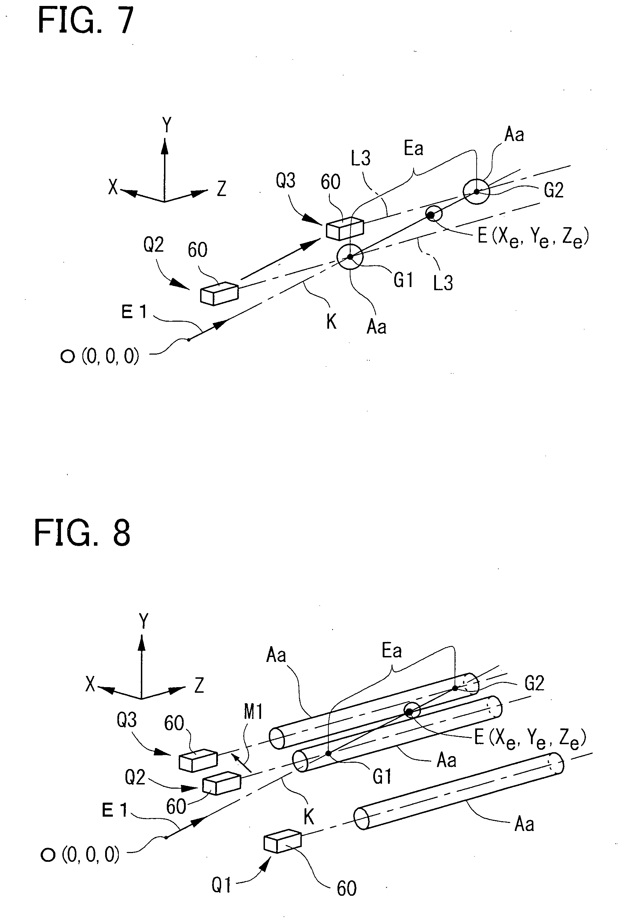

The CPU 71 for instance moves the anterior segment photographing optical system 60 to the position Q2 and dispose the alignment allowable region Aa on the straight line K. The CPU 71 further moves the anterior segment photographing optical system 60 in a direction based on the straight line K while determining whether the bright spot is detected or not on the anterior segment image Ia. For instance, the anterior segment photographing optical system 60 may be moved from the position Q2 toward a position Q3 as shown in FIG. 7. Herein, the position Q3 is for example a location causing the position G2 farthest from the origin O of the measurable range Ea to coincide with the gravity point of the alignment allowable region Aa.

The CPU 71 for instance moves the anterior segment photographing optical system 60 in the direction E1 along the straight line K so that the alignment allowable region Aa includes at least part of the straight line K. This enables positioning the examinee's eye E in the alignment allowable region Aa during movement from the position Q2 to the position Q3. When the examinee's eye E enters the alignment allowable region Aa, the CPU 71 can perform alignment of the optometry unit 2 with the examinee's eye E based on the anterior segment image Ia photographed by the anterior segment photographing optical system 60.

When the optometry unit 2 is to be moved, this movement may be started from a position at which a relative distance (Z) between the optometry unit 2 and the face support unit 9 is sufficiently large. This can prevent the optometry unit 2 from colliding against an examinee.

When the anterior segment photographing optical system 60 is to be moved from the position Q1 to the position Q2, for instance, it may be moved so that the alignment allowable region Aa and the straight line K overlap in the shortest time, it may be moved at a short total movement distance, or it may be moved in an arbitrary movement direction. For instance, the CPU 71 may be configured to move the alignment allowable region Aa in a direction perpendicular to the straight line K so that the alignment allowable region Aa is moved by a shortest distance to the straight line K.

During movement from the position Q2, this movement has only to be performed so that the alignment allowable region Aa includes a region on the straight line K. That movement direction is not limited to the direction E1 along the straight line K. For instance, when the alignment allowable region Aa is sufficiently wide in the Z direction with respect to the measurable range Ea, the anterior segment photographing optical system 60 can be moved in an X-Y direction M1 from the position Q2 to the position Q3. Herein, the position Q3 is for example a position at which the position G2 distant from the origin O within the measurable range Ea is included in the alignment allowable region Aa, and a Z-coordinate of the position Q3 is equal to that of Q2.

The CPU 71 may be configured to update this direction E1 of the examinee's eye E as needed. In the process of alignment, the visual line of the examinee's eye E may change in some cases. In a case where a fixation target is not visible to an examinee, for example, his/her visual line is not fixed. A coordinate $(x_e, y_e)$ of a pupil on the anterior segment image Ia changes depending on the direction of the visual line of the examinee's eye E. As a result, the direction E1 of the examinee's eye E also changes. Accordingly, the examinee's eye E in a measurable state (a fixated state) may not be present in the direction E1 of the examinee's eye E determined at the start of measurement. Therefore the CPU 71 may be configured to update the direction E1 of the examinee's eye E in association with time change and movement of the apparatus.

(S7: Alignment with bright spot)

The CPU 71 controls the drive unit 4 based on the position of the bright spot detected in step S1 to adjust the measurement axis L1 of the optometry unit 2 to the examinee's eye E.

(S8: Determination of alignment completion)

Upon completion of driving for alignment, the CPU 71 determines whether the alignment has been completed or not. For instance, this determination is made, referring to the image produced by the anterior segment photographing optical system 60, on whether a corneal reflection image falls within the alignment allowable range.

(S9: Measurement)

When the CPU 71 determines in step S8 that the alignment has been completed, the CPU 71 causes the optometry unit 2 to start measurement of the examinee's eye E. For instance, the optometry unit 2 measures eye refractive power of the examinee's eye E. Needless to say, not limited to measurement of the refractive power, various measurement, photographing, and so on are executed according to the types of optometry units.

In the ophthalmic apparatus 1 of the present example configured as above, the optometry unit 2 is moved to the alignment allowable region Aa based on the direction E1 of the examinee's eye E obtained from the face image Pi photographed by the face photographing unit 3. Accordingly, for instance, the optometry unit 2 can be automatically moved to a position allowing alignment with respect to the examinee's eye E even without using a telecentric optical system, a stereo camera, and others as the photographing optical system 3A of the face photographing unit 3.

In a configuration described in the present example that the face photographing unit 3 is provided separately from a measurement system (e.g., the optometry unit 2 and others) as in the present example, the optometry unit 2 can be automatically moved to an alignment allowing position in an independent manner from the measurement system. Thus, this configuration is easily applicable to various types of ophthalmic apparatuses.

In the aforementioned examples, the direction E1 of the examinee's eye E seen from the face photographing unit 3 is obtained. However, the present disclosure is not limited thereto. For instance, the CPU 71 may also be configured to calculate a direction of the corner of the eye seen from the face photographing unit 3. For instance, three-dimensional relative positions of the corner and the pupil of the eye are determined in advance and information on those positions is stored in the apparatus. In this case, the CPU 71 detects the eye's corner on the face image Pi and calculates the direction of the eye's corner in the same manner as above. The CPU 71 obtains the direction E1 of the examinee's eye E seen from the face photographing unit 3 based on the restrictive condition obtained from the direction of the eye's corner and the three-dimensional relative positions of the eye's corner and the pupil. By utilizing this, the CPU 71 may perform the aforementioned alignment. The position change of the corner of the eye is less caused by visual line change, so that the optometry unit 2 can be moved in a stable direction even when the visual line changes.

The CPU 71 may be configured to obtain the face image Pi including both, right and left, eyes of the examinee photographed by the face photographing unit 3. In this case, the CPU 71 may be configured to calculate both directions of those right and left eyes seen from the face photographing unit 3 and store the calculated directions in the storage unit 74 and others. When measurement on one of the examinee's eyes has been completed and alignment to the other eye is to be performed, the right eye and the left eye may also be changed over based on the position of the eye having been subjected to measurement and the direction of the other eye seen from the face photographing unit 3. For instance, the CPU 71 may be configured to move the optometry unit 2 from the position where one of the examinee's eyes has been measured toward the straight line on which the other examinee's eye is present. This enables smooth change-over of the right eye and the left eye.

A method for detecting the examinee's eye E from an image may include various image processing methods by for example detecting a pupil by infrared photographing, detecting an edge of a luminance value, and other techniques. In the case of photographing the face of an examinee with infrared light, his/her skin shows up white and the pupil shows up black. Thus, the CPU 71 may be configured to detect, as a pupil, a circular black (low luminance) portion from an infrared image obtained by the infrared photography. With the aforementioned method, the CPU 71 detects the examinee's eye E from the face image Pi and acquires the information on the two-dimensional position.

In the present example, the alignment target is detected on the anterior segment image Ia photographed by the anterior segment photographing optical system 60 to perform final alignment. However, the present disclosure is not limited thereto. For instance, the CPU 71 may be configured to perform final alignment from a pupil position on the anterior segment image la, contrast, edge, and others.

**Claims**

1. An ophthalmic apparatus (1) for examining an eye (E) of an examinee, comprising:

   optometry means (2) configured to examine the examinee's eye;
   drive means (4) for relatively moving the optometry means three-dimensionally with respect to the examinee's eye;
   first photographing means (3) configured to photograph a face of the examinee including at least one of right and left eyes of the examinee; and
   control means (70) configured to control the drive means,
   wherein
   the control means is further configured to restrict a drive range of the drive means based on a two-dimensional position ($P(x_e, y_e)$) of the examinee's eye on a first image (Pi) photographed by the first photographing means, the two-dimensional position being specified by image processing on the first image, and information on the first photographing means including positional information of the first photographing means, the control means (70) is further configured to restrict the drive range of the drive means based on a three-dimensional relative direction between the examinee's eye (E) and the first photographing means, the three-dimensional relative direction being calculated based on the two-dimensional position and the information on the first photographing means, and
   the optometry means (2) is further provided with second photographing means (60) configured to observe an anterior segment of the examinee's eye (E), **characterized in that**
   the control means (70) is further configured such that:

   when a positional relationship between the examinee's eye and the optometry means is obtained by image processing on a second image (Ia) photographed by the second photographing means, the control means (70) controls the drive means (4) based on the positional relationship to perform alignment of the optometry means (2) with respect to the examinee's eye, or
   when the positional relationship is not obtained from the second image, the control means (70) restricts the drive range of the drive means based on the two-dimensional position of the examinee's eye on the first image specified by image processing on the first image (Pi) and the information on the first photographing means,
   the control means (70) is configured to calculate a straight line (K) passing through a position of the first photographing means (3) and having a directional vector defined by the three-dimensional relative direction, and restrict the drive range so that at least part of the straight line is included in an alignment allowable region (Aa) by the second photographing means (60), and
   the control means (70) is further configured to control the drive means (4) within the restricted drive range to move the optometry means (2) to the position where the examinee's eye is included in the alignment allowable region provided by the second photographing means (60).

2. The ophthalmic apparatus of claim 1, wherein the control means (70) is configured to control the drive means to move the alignment allowable region by the second photographing means (60) along the straight line.

3. The ophthalmic apparatus of one of claims 1 to 2, wherein the control means (70) is configured to update a restrictive condition of the drive range based on a new first image (Pi) photographed by the first photographing means (3).

4. The ophthalmic apparatus of one of claims 1 to 3, wherein the first photographing means (3) is provided in the optometry means (2) and is moved together with the second photographing means (60) by the drive means (4).

5. The ophthalmic apparatus of one of claims 1 to 3 further comprising face support means (9) for supporting the face of the examinee,
   wherein a relative positional relationship between the face support means and the first photographing means (3) is constant.

6. The ophthalmic apparatus of one of claims 1 to 5, wherein the control means (70) is further configured to detect a pupil of the examinee's eye (E) from the first image (Pi) photographed by the first photographing means (3) and restrict the drive range of the drive means based on a two-dimensional position of the pupil on the first image and the information on the first photographing means.

7. The ophthalmic apparatus of one of claims 1 to 6, further comprising a target light source (50) for projecting an alignment target onto a cornea of the examinee's eye,
wherein the control means (70) is configured to detect a position of the alignment target from the second image (Ia) photographed by the second photographing means (60) and perform alignment of the optometry means (2) with respect to the examinee's eye based on the position of the alignment target.

8. A control program for an ophthalmic apparatus (1) for examining an eye (E) of an examinee, the program being executed in the ophthalmic apparatus,
wherein the program is executed by a processor (70) of the ophthalmic apparatus to cause the ophthalmic apparatus to perform:

a photographing step of causing first photographing means (3) to photograph a face of the examinee, including at least one of right and left eyes of the examinee;
a drive step of relatively moving the optometry means three-dimensionally with respect to the examinee's eye based on a two-dimensional position of the examinee's eye on a first image (Pi) photographed in the photographing step, the two-dimensional position being specified by image processing on the first image, and information on the photographing means including positional information of the first photographing means;
an optometry step of examining the examinee's eye, and
calculating step of a three-dimensional relative direction between the examinee's eye (E) and the first photographing means based on the two-dimensional position and the information on the first photographing means, **characterized in that**
a drive step of relatively moving the optometry means three-dimensionally with respect to the examinee's eye based on a positional relationship between the examinee's eye and the optometry means obtained by image processing on a second image (Ia) photographed by a second photographing means (60) provided in the optometry means (2) and configured to observe an anterior segment of the examinee's eye (E), when the positional relationship between the examinee's eye and the optometry means is obtained by image processing on the second image (Ia), or
a restricting step of a drive range of three-dimensional relative movement of the optometry means with respect to the examinee's eye based on the two-dimensional position of the examinee's eye on the first image specified by image processing on the first image (Pi) and the information on the first photographing means, when the positional relationship between the examinee's eye and the optometry means is not obtained from the second image,
a calculating step of a straight line (K) passing through a position of the first photographing means (3) and having a directional vector defined by the three-dimensional relative direction,
a restricting step of the drive range so that at least part of the straight line is included in an alignment allowable region (Aa) by the second photographing means (60), and
a drive step of relatively moving the optometry means three-dimensionally with respect to the examinee's eye within the restricted drive range to the position where the examinee's eye is included in the alignment allowable region provided by the second photographing means (60).

**Patentansprüche**

1. Ophthalmisches Gerät (1) zum Untersuchen eines Auges (E) eines Prüflings, aufweisend:

ein optometrisches Mittel (2), das dazu ausgebildet ist, das Auge des Prüflings zu untersuchen;
ein Antriebsmittel (4) zum dreidimensionalen relativen Bewegen des optometrischen Mittels bezüglich des Auges des Prüflings;
ein erstes fotografierendes Mittel (3), das dazu konfiguriert ist, ein Gesicht des Prüflings einschließlich zumindest einem von einem rechten oder linken Auge des Prüflings zu fotografieren; und
ein Steuerungsmittel (70), das dazu konfiguriert ist, das Antriebsmittel zu steuern, wobei
das Steuerungsmittel ferner dazu konfiguriert ist, einen Antriebsbereich des Antriebsmittels basierend auf einer zweidimensionalen Position $(P(x_e, y_e))$ des Auges des Prüflings auf einem durch das erste fotografierende Mittel fotografierten ersten Bild (Pi), wobei die zweidimensionale Position durch eine Bildbearbeitung des ersten Bilds spezifiziert ist, und einer Information in dem ersten fotografierenden Mittel einschließlich einer Positionsinformation des ersten fotografierenden Mittels einzuschränken, das Steuerungsmittel (70) ferner dazu konfiguriert ist, den Antriebsbereich des Antriebsmittels basierend auf einer dreidimensionalen relativen Richtung zwischen dem Auge (E) des Prüflings und dem ersten fotografierenden Mittel einzuschränken, wobei die dreidimensionale relative Richtung basierend auf der zweidimensionalen Position und der Information in dem ersten fotografierenden Mittel berechnet wird, und

das optometrische Mittel (2) ferner mit einem zweiten fotografierenden Mittel (60) versehen ist, das dazu konfiguriert ist, einen vorderen Abschnitt des Auges (E) des Prüflings zu beobachten, **dadurch gekennzeichnet, dass**

das Steuerungsmittel (70) ferner so konfiguriert ist, dass:

wenn ein Positionsverhältnis zwischen dem Auge des Prüflings und dem optometrischen Mittel durch eine Bildbearbeitung eines durch das zweite fotografierende Mittel fotografierten zweiten Bilds (la) erhalten wird, das Steuerungsmittel (70) das Antriebsmittel (4) basierend auf dem Positionsverhältnis steuert, um ein Ausrichten des optometrischen Mittels (2) bezüglich des Auges des Prüflings durchzuführen, oder

wenn das Positionsverhältnis nicht aus dem zweiten Bild erhalten wird, das Steuerungsmittel (70) den Antriebsbereich des Antriebsmittels basierend auf der durch eine Bildbearbeitung des ersten Bilds (Pi) spezifizierten zweidimensionalen Position des Auges des Prüflings und der Information in dem ersten fotografierenden Mittel einschränkt,

das Steuerungsmittel (70) dazu konfiguriert ist, eine gerade Linie (K), die durch eine Position des ersten fotografierenden Mittels (3) geht und einen durch die dreidimensionale relative Richtung definierten Richtungsvektor hat, zu berechnen, und den Antriebsbereich einzuschränken, so dass zumindest ein Teil der geraden Linie in einem durch das zweite fotografierende Mittel (60) zulässigen Ausrichtungsbereich (Aa) ist, und

das Steuerungsmittel (70) ferner dazu konfiguriert ist, das Antriebsmittel (4) innerhalb des eingeschränkten Antriebsbereichs zu steuern, um das optometrische Mittel (2) zu der Position zu bewegen, in der das Auge des Prüflings in dem von dem zweiten fotografierenden Mittel (60) bereitgestellten zulässigen Ausrichtungsbereich enthalten ist.

2. Ophthalmisches Gerät von Anspruch 1, wobei das Steuerungsmittel (70) dazu konfiguriert ist, das Antriebsmittel zu steuern, um den durch das zweite fotografierende Mittel (60) zulässigen Ausrichtungsbereich entlang der geraden Linie zu bewegen.

3. Ophthalmisches Gerät von einem der Ansprüche 1 bis 2, wobei das Steuerungsmittel (70) dazu konfiguriert ist, eine einschränkende Bedingung des Antriebsbereichs basierend auf einem durch das erste fotografierende Mittel (3) fotografierten neuen ersten Bild (Pi) zu aktualisieren.

4. Ophthalmisches Gerät von einem der Ansprüche 1 bis 3, wobei das erste fotografierende Mittel (3) in dem optometrischen Mittel (2) vorgesehen ist und zusammen mit dem zweiten fotografierenden Mittel (60) durch das Antriebsmittel (4) bewegt wird.

5. Ophthalmisches Gerät von einem der Ansprüche 1 bis 3, das ferner ein Gesichtslagerungsmittel (9) zum Lagern des Gesichts des Prüflings aufweist,

wobei ein relatives Positionsverhältnis zwischen dem Gesichtslagerungsmittel und dem ersten fotografierenden Mittel (3) konstant ist.

6. Ophthalmisches Gerät von einem der Ansprüche 1 bis 5, wobei das Steuerungsmittel (70) ferner dazu konfiguriert ist, eine Pupille des Auges (E) des Prüflings von dem durch das erste fotografierende Mittel (3) fotografierten ersten Bild (Pi) zu erfassen, und den Antriebsbereich des Antriebmittels basierend auf einer zweidimensionalen Position der Pupille auf dem ersten Bild und der Information in dem ersten fotografierenden Mittel einzuschränken.

7. Ophthalmisches Gerät von einem der Ansprüche 1 bis 6, das ferner eine Ziellichtquelle (50) zum Projizieren eines Ausrichtungsziels auf eine Hornhaut des Auges des Prüflings aufweist,

wobei das Steuerungsmittel (70) dazu konfiguriert ist, eine Position des Ausrichtungsziels von dem durch das zweite fotografierende Mittel (60) fotografierten zweiten Bild (la) zu erfassen und ein Ausrichten des optometrischen Mittels (2) bezüglich des Auges des Prüflings basierend auf der Position des Ausrichtungsziels durchzuführen.

**8.** Steuerungsprogramm für ein ophthalmisches Gerät (1) zum Untersuchen eines Auges (E) eines Prüflings, wobei das Programm in dem ophthalmischen Gerät ausgeführt wird,
wobei das Programm durch einen Prozessor (70) des ophthalmischen Geräts ausgeführt wird um das ophthalmische Gerät zu veranlassen, auszuführen:

einen Fotografierschritt eines Veranlassens eines ersten fotografierenden Mittels (3), ein Gesicht eines Prüflings einschließlich zumindest einem von einem rechten oder linken Auge des Prüflings zu fotografieren;
einen Antriebsschritt eines dreidimensionalen relativen Bewegens des optometrischen Mittels bezüglich des Auges des Prüflings basierend auf einer zweidimensionalen Position des Auges des Prüflings auf einem in dem Fotografierschritt fotografierten ersten Bild (Pi), wobei die zweidimensionale Position durch eine Bildbearbeitung des ersten Bilds spezifiziert wird, und einer Information in dem fotografierenden Mittels einschließlich einer Positionsinformation des ersten fotografierenden Mittels,
einen optometrischen Schritt eines Prüfens des Auges des Prüflings, und
einen Berechnungsschritt einer dreidimensionalen relativen Richtung zwischen dem Auge (E) des Prüflings und dem ersten fotografierenden Mittel basierend auf der zweidimensionalen Position und der Information in dem ersten fotografierenden Mittel,
**gekennzeichnet durch**
einen Antriebsschritt eines dreidimensionalen relativen Bewegens des optometrischen Mittels bezüglich des Auges des Prüflings basierend auf einem Positionsverhältnis zwischen dem Auge des Prüflings und dem optometrischen Mittel, das durch Bildbearbeitung eines zweiten Bilds (la), das durch ein in dem optometrischen Mittel (2) vorgesehenes und dazu konfiguriertes, einen vorderen Abschnitt des Auges (E) des Prüflings zu beobachten, zweites fotografierendes Mittel (60) fotografiert wird, erhalten wird, wenn das Positionsverhältnis zwischen dem Auge des Prüflings und dem optometrischen Mittel durch Bildbearbeitung des zweiten Bilds (la) erhalten wird, oder
einen Einschränkungsschritt eines Antriebsbereichs einer dreidimensionalen relativen Bewegung des optometrischen Mittels bezüglich des Auge des Prüflings basierend auf der durch eine Bildbearbeitung des ersten Bilds (Pi) spezifizierten zweidimensionalen Position des Auges des Prüflings auf dem ersten Bild und der Information in dem ersten fotografierenden Mittel, wenn das Positionsverhältnis zwischen dem Auge des Prüflings und dem optometrischen Mittel nicht aus dem zweiten Bild erhalten wird,
einen Berechnungsschritt einer geraden Linie (K), die durch eine Position des ersten fotografierenden Mittels (3) durchgeht und einen Richtungsvektor hat, der durch die dreidimensionale relative Richtung definiert ist,
einen Einschränkungsschritt eines Antriebsbereichs, so dass zumindest ein Teil der geraden Linie in einem durch das zweite fotografierende Mittel (60) zulässigen Ausrichtungsbereich (Aa) enthalten ist, und
einen Antriebsschritt eines dreidimensionalen relativen Bewegens des optometrischen Mittels bezüglich des Auges des Prüflings innerhalb des eingeschränkten Antriebsbereichs zu der Position, in der das Auge des Prüflings in dem durch das zweite fotografierende Mittel (60) bereitgestellten zulässigen Ausrichtungsbereich enthalten ist.

**Revendications**

**1.** Appareil ophtalmique (1) permettant d'examiner un œil (E) d'une personne examinée, comprenant :

des moyens optométriques (2) configurés pour examiner l'œil de la personne examinée ;
des moyens d'entraînement (4) pour déplacer relativement les moyens optométriques de manière tridimensionnelle par rapport à l'œil de la personne examinée ;
des premiers moyens photographiques (3) configurés pour photographier un visage de la personne examinée incluant au moins l'un de l'œil droit et de l'œil gauche de la personne examinée ; et
des moyens de commande (70) configurés pour commander les moyens d'entraînement,
dans lequel
les moyens de commande sont en outre configurés pour limiter une plage d'entraînement des moyens d'entraînement sur la base d'une position bidimensionnelle ($P(x_e, y_e)$) de l'œil de la personne examinée sur une première image (Pi) photographiée par les premiers moyens photographiques, la position bidimensionnelle étant spécifiée par un traitement d'image sur la première image, et des informations relatives aux premiers moyens photographiques incluant des informations positionnelles des premiers moyens photographiques, les moyens de commande (70) étant en outre configurés pour limiter la plage d'entraînement des moyens d'entraînement sur la base d'une direction relative tridimensionnelle entre l'œil (E) de la personne examinée et les premiers moyens photographiques, la direction relative tridimensionnelle étant calculée sur la base de la position bidimensionnelle et des informations relatives aux premiers moyens photographiques, et
les moyens optométriques (2) sont en outre pourvus de seconds moyens photographiques (60) configurés pour observer un segment antérieur de l'œil (E) de la personne examinée, **caractérisé en ce que**
les moyens de commande (70) sont en outre configurés de sorte que :

lorsqu'une relation positionnelle entre l'œil de la personne examinée et les moyens optométriques est obtenue par un traitement d'image sur une seconde image (Ia) photographiée par les seconds moyens photographiques, les moyens de commande (70) commandent les moyens d'entraînement (4) sur la base de la relation positionnelle pour réaliser un alignement des moyens optométriques (2) par rapport à l'œil de la personne examinée, ou
lorsque la relation positionnelle n'est pas obtenue à partir de la seconde image, les moyens de commande (70) limitent la plage d'entraînement des moyens d'entraînement sur la base de la position bidimensionnelle de l'œil de la personne examinée sur la première image spécifiée par un traitement d'image sur la première image (Pi) et des informations relatives aux premiers moyens photographiques,
les moyens de commande (70) sont configurés pour calculer une ligne droite (K) passant par une position des premiers moyens photographiques (3) et ayant un vecteur directionnel défini par la direction relative tridimensionnelle, et limiter la plage d'entraînement de sorte qu'au moins une partie de la ligne droite soit incluse dans une région d'alignement admissible (Aa) par les seconds moyens photographiques (60), et
les moyens de commande (70) sont en outre configurés pour commander les moyens d'entraînement (4) à l'intérieur de la plage d'entraînement limitée afin de déplacer les moyens optométriques (2) à la position à laquelle l'œil de la personne examinée est inclus dans la région d'alignement admissible fournie par les seconds moyens photographiques (60).

2. Appareil ophtalmique selon la revendication 1, dans lequel les moyens de commande (70) sont configurés pour commander les moyens d'entraînement afin de déplacer la région d'alignement admissible par les seconds moyens photographiques (60) le long de la ligne droite.

3. Appareil ophtalmique selon la revendication 1 ou 2, dans lequel les moyens de commande (70) sont configurés pour mettre à jour une condition limitative de la plage d'entraînement sur la base d'une nouvelle première image (Pi) photographiée par les premiers moyens photographiques (3).

4. Appareil ophtalmique selon l'une des revendications 1 à 3, dans lequel les premiers moyens photographiques (3) sont prévus dans les moyens optométriques (2) et sont déplacés solidairement avec les seconds moyens photographiques (60) par les moyens d'entraînement (4).

5. Appareil ophtalmique selon l'une des revendications 1 à 3, comprenant en outre des moyens de soutien de visage (9) permettant de soutenir le visage de la personne examinée,
dans lequel une relation positionnelle relative entre les moyens de soutien de visage et les premiers moyens photographiques (3) est constante.

6. Appareil ophtalmique selon l'une des revendications 1 à 5, dans lequel les moyens de commande (70) sont en outre configurés pour détecter une pupille de l'œil (E) de la personne examinée à partir de la première image (Pi) photographiée par les premiers moyens photographiques (3) et limiter la plage d'entraînement des moyens d'entraînement sur la base d'une position bidimensionnelle de la pupille sur la première image et des informations relatives aux premiers moyens photographiques.

7. Appareil ophtalmique selon l'une des revendications 1 à 6, comprenant en outre une source lumineuse cible (50) pour projeter une cible d'alignement sur une cornée de l'œil de la personne examinée,
dans lequel les moyens de commande (70) sont configurés pour détecter une position de la cible d'alignement à partir de la seconde image (Ia) photographiée par les seconds moyens photographiques (60) et réaliser un alignement des moyens optométriques (2) par rapport à l'œil de la personne examinée sur la base de la position de la cible d'alignement.

8. Programme de commande pour un appareil ophtalmique (1) permettant d'examiner un œil (E) d'une personne examinée, le programme étant exécuté dans l'appareil ophtalmique,
dans lequel le programme est exécuté par un processeur (70) de l'appareil ophtalmique pour amener l'appareil ophtalmique à réaliser :

une étape de photographie pour amener des premiers moyens photographiques (3) à photographier un visage de la personne examinée, incluant au moins l'un de l'œil droit et de l'œil gauche de la personne examinée ;

une étape d'entraînement pour déplacer relativement les moyens optométriques de manière tridimensionnelle par rapport à l'œil de la personne examinée sur la base d'une position bidimensionnelle de l'œil de la personne examinée sur une première image (Pi) photographiée à l'étape de photographie, la position bidimensionnelle étant spécifiée par un traitement d'image sur la première image, et des informations relatives aux moyens photographiques incluant des informations positionnelles des premiers moyens photographiques ;

une étape d'optométrie pour examiner l'œil de la personne examinée ; et

une étape de calcul d'une direction relative tridimensionnelle entre l'œil (E) de la personne examinée et les premiers moyens photographiques sur la base de la position bidimensionnelle et des informations relatives aux premiers moyens photographiques,

**caractérisé par**

une étape d'entraînement pour déplacer relativement les moyens optométriques de manière tridimensionnelle par rapport à l'œil de la personne examinée sur la base d'une relation positionnelle entre l'œil de la personne examinée et les moyens optométriques qui est obtenue par un traitement d'image sur une seconde image (Ia) photographiée par des seconds moyens photographiques (60) prévus dans les moyens optométriques (2) et configurés pour observer un segment antérieur de l'œil (E) de la personne examinée, lorsque la relation positionnelle entre l'œil de la personne examinée et les moyens optométriques est obtenue par un traitement d'image sur la seconde image (Ia), ou

une étape de limitation d'une plage d'entraînement d'un mouvement relatif tridimensionnel des moyens optométriques par rapport à l'œil de la personne examinée sur la base de la position bidimensionnelle de l'œil de la personne examinée sur la première image qui est spécifiée par un traitement d'image sur la première image (Pi) et des informations relatives aux premiers moyens photographiques, lorsque la relation positionnelle entre l'œil de la personne examinée et les moyens optométriques n'est pas obtenue à partir de la seconde image,

une étape de calcul d'une ligne droite (K) passant par une position des premiers moyens photographiques (3) et ayant un vecteur directionnel défini par la direction relative tridimensionnelle,

une étape de limitation de la plage d'entraînement de sorte qu'au moins une partie de la ligne droite soit incluse dans une région d'alignement admissible (Aa) par les seconds moyens photographiques (60), et

une étape d'entraînement pour déplacer relativement les moyens optométriques de manière tridimensionnelle par rapport à l'œil de la personne examinée à l'intérieur de la plage d'entraînement limitée à la position à laquelle l'œil de la personne examinée est inclus dans la région d'alignement admissible fournie par les seconds moyens photographiques (60).

# FIG. 1

# FIG. 2

FIG. 3

EP 3 150 111 B1

# FIG. 4

```
                        START

                          │
                          ▼
            ┌──────────────────────────┐
            │  ANTERIOR SEGMENT        │
            │  PHOTOGRAPHING UNIT:     │── S1
            │  DETECTION OF BRIGHT     │
            │  SPOT                    │
            └──────────────────────────┘
                          │
                          ▼
                        S2
                   ╱         ╲
                  ╱ IS BRIGHT ╲        No
                 ╱   SPOT      ╲──────────────┐
                 ╲  PRESENT?   ╱               │
     S7           ╲          ╱                 │
                   ╲        ╱                  ▼
                      │ Yes                 S3
                      ▼                  ┌──────────────────────────┐
         ┌──────────────────┐           │  FACE PHOTOGRAPHING UNIT:│
         │   ALIGNMENT      │           │  DETECTION OF PUPIL      │
         │  WITH BRIGHT SPOT│           └──────────────────────────┘
         └──────────────────┘                     │
                      │                            ▼
                      │                          S4
                      │                      ╱        ╲
                      │                     ╱ IS PUPIL ╲     No
                      │                    ╱  PRESENT?  ╲────────┐
                      │                     ╲          ╱         │
                      │                      ╲        ╱          │
                      │                         │ Yes            │
                      │                         ▼                │
                      │           ┌──────────────────────────────┐
                      │    S5     │ DERIVATION OF DIRECTION OF    │
                      │           │ EXAMINEE'S EYE SEEN FROM      │
                      │           │ FACE PHOTOGRAPHING UNIT       │
                      │           └──────────────────────────────┘
                      │                         │
                      │                         ▼
                      │    S6     ┌──────────────────────────────┐
                      │           │ MOVEMENT BASED ON            │
     S8               │           │ DIRECTION OF EXAMINEE'S EYE  │
                      ▼           └──────────────────────────────┘
                 ╱         ╲                    │                │
                ╱ ALIGNMENT ╲    No             │                │
                ╲   OK?     ╱───────────────────┴────────────────┘
                 ╲         ╱
                    │ Yes
                    ▼
         ┌──────────────────┐
         │   MEASUREMENT    │── S9
         └──────────────────┘
                    │
                    ▼
                  END
```

## FIG. 5

## FIG. 6

# FIG. 7

# FIG. 8

**REFERENCES CITED IN THE DESCRIPTION**

**Patent documents cited in the description**

- EP 2085023 A1
- JP 2013066760 A

- JP H101998216089 B